# EUROPEAN PATENT APPLICATION

(11) **EP 1 561 467 A1**
(43) Date of publication of application: **10.08.2005**
(21) Application number: 03811090.4
(22) Date of filing: 10.11.2003
(51) Int. Cl.: A61K 31/79, A61K 33/18, A61K 47/24, A61K 47/36, A61P 17/02

(54) **COMPOSITION FOR RESTORING DAMAGED SKIN**

(30) Priority: 11.11.2002 JP 2002326535
(71) Applicant: Kowa Co., Ltd., Naka-ku, Nagoya-shi, Aichi 460-8625 (JP); Teika Pharmaceutical Co., Ltd., Toyama-shi, Toyama 930-0982 (JP)
(72) Inventor: NISHIMURA, Masahiro, 1151-1-3, Hara, Shizuoka 410-0312 (JP); NITO, Shihomi, 166-307, Ohzuwa, Shizuoka 410-0873 (JP); INAGI, Toshio, 6-10-203, Nishiwakacho, Shizuoka 411-0038 (JP); KIMURA, Takahito, 81, Taroumaru 2-ku, Toyama 939-8076 (JP)
(74) Representative: Hartz, Nikolai F., Dr.
(86) International application number: PCT/JP2003/014251
(87) International publication number: WO 2004/043473

(57) **Abstract**

The present invention provides a composition for repairing injured skin containing saccharide and povidone-iodine of the present invention having characteristices of suppressing time-dependent increase of consistency, easy to use, soft preparation, and superior applicability to a deep wounded lesion and a granulation tissue.

The composition for repairing injured skin comprises 50 to 90% by weight of saccharide, 0.5 to 10% by weight of povidone-iodine, 0.1 to 20% by weight of water and 0.01 to 10% by weight of phospholipid.

## Description

### Technical Field

The present invention relates to a composition for repairing injured skin with suppressed time-dependent increasing consistency of a pharmaceutical preparation comprising saccharide and povidone-iodine (polyvinylpyrrolidone-iodine complex, poly[(2-oxopyrrolidin-1-yl)ethylene]iodine).

### Background Art

Since a saccharide such as white soft sugar has a wound healing action and a granulation tissue forming action, a saccharide mixed with povidone-iodine is used for the pharmaceutical preparation for healing injury of the skin such as bedsore and skin ulcer [e.g. refer to JP-B-01-032210, JP-B-06-017299, "SouthernMedical Journal", 1981, 74(11), 1329-1335, and "Byoin Yakugaku", 1984, 10(5), 315-322].

However, since a composition for repairing injured skin (hereinafter, sometimes designated as ointment preparation) containing saccharide and povidone-iodine consists of sugar for the most part, it has an intrinsic property of saccharide to harden in a time-dependent manner. Consequently, when it is used in the medical care field by spreading onto a gauze, it is necessary to use after stirring and softening the hardened ointment preparation, and complicated procedure and time are required for co-medicals.

In order to improve such disadvantages of time-dependent increasing consistency, a method for formulating a plurality of powdery saccharides different in their average particle sizes (JP-A-11-171779), a method for formulating non-volatile solvent and hydroxylated lower alkylamine (JP-A-11-228421), etc. are known, however said methods are still not satisfactory.

A powdered formulation has also disadvantages such that it is difficult to apply in an affected part, and further scatter in the air causes contamination of the ambiance.

Another method for reducing the consistency of the composition for repairing injured skin containing saccharide and povidone-iodine includes preparations with a reduced amount of the polymer base to be blended or without blending it, but it is not preferable due to separation of a composition of the preparation in a time-dependent manner.

Consequently, development of a composition for repairing injured skin containing saccharide and povidone-iodine without time-dependent increase in consistency and hardening is required.

### Disclosure of the Invention

An object of the present invention is to provide a composition for repairing injured skin with suppressed time-dependent increase of consistency of a pharmaceutical preparation comprising saccharide and povidone-iodine.

The inventors of the present invention have continued extensive studies considering the above disadvantages, and as a result, surprisingly found that a composition for repairing injured skin containing saccharide and povidone-iodine with suppressed time-dependent increase of consistency and with superior stability can be obtained by formulating a certain amount of phospholipid in a formulation containing saccharide, povidone-iodine and water, and completed the present invention.

Thus, an aspect of the present invention is to provide a composition for repairing injured skin comprising 50 to 90% by weight of saccharide, 0.5 to 10% by weight of povidone-iodine, 0.1 to 20% by weight of water and 0.01 to 10% by weight of phospholipid.

Said composition for repairing injured skin containing saccharide and povidone-iodine of the present invention is easy to use due to suppressed time-dependent increase of consistency and suitable for application to a deep wound and a granulation tissue surface due to soft pharmaceutical preparation.

### Best Mode for Carrying Out the Invention

Saccharide used in the present invention includes non-reducing sugar and reducing sugar such as white soft sugar (including purified white soft sugar), glucose, honey and molasses, and white soft sugar is preferable.

Amount of the saccharide to be blended is 50 to 90% by weight, preferably 60 to 80% by weight, and most preferably 70% by weight to the total pharmaceutical preparation.

Amount of the povidone-iodine to be blended is 0.5 to 10% by weight, preferably 1 to 7% by weight, and most preferably 2 to 6% by weight to the total pharmaceutical preparation.

Amount of water to be added is 0.1 to 20% by weight, preferably 0.3 to 15% by weight, and most preferably 0.5 to 12% by weight to the total pharmaceutical preparation.

Phospholipid to be used in the present invention is a conjugated lipid containing phosphate residue such as natural phospholipids, synthetic phospholipids and hydrogenated phospholipids, in which a naturally occurring phospholipid is hydrogenated.

The natural phospholipids include phosphatidylcholine, phosphatidylethanolamine, phosphatidylserine, phosphatidylinositol, lysophosphatidylcholine, sphingomyelin, egg yolk lecithin, soybean lecithin and phospholipid extracted from microorganisms such as E. coli. Commercially available products are, for example, COATSOME NC-50 (NOF) and presome (Nippon Seika, Co. Ltd.).

The synthetic phospholipids include dioleoyl-phosphatidylcholine, dilauroyl-phosphatidylcholine, dimyristoyl-phosphatidylcholine, dipalmitoyl-phosphatidylcholine, distearoyl-phosphatidylcholine, and palmitoyl-oleoyl-phosphatidylcholine. Commercially available products are, for example, COATSOME MC-2020, COATSOME MC-4040, COATSOMEMC-6060,COATSOME MC-8080,COATSOME-MC-8181 and COATSOME MC-6081 (NOF).

The hydrogenated phospholipids include hydrogenated soybean phospholipid, hydrogenated egg yolk phospholipid, hydrogenated phosphatidylcholine and hydrogenated phosphatidylserine. Commercially available products are Lecinol S-10, Lecinol S-10E, Lecinol S-10M, Lecinol S-10EX, Lecinol S-PIE (Nikko Chemicals, Co.), COATSOME NC-21 (NOF), Phospholipon and Phosal (Aventis).

Among these phospholipids, natural phospholipids or hydrogenated phospholipids are preferable.

In the present invention, phospholipid can be used alone or in combination of two or more kinds, and amount thereof to be blended is 0.01 to 10% by weight, preferably 0.01 to 7% by weight, more preferably 0.05 to 5% by weight, and most preferably 0.1 to 5% by weight to the total pharmaceutical preparation. If the amount of phospholipid is below 0.01% by weight, asufficient stabilizing effect on consistency can not be obtained. The amount of phospholipid above 10% by weight is not preferable, because stability of the preparation is decreased.

The pH of the composition for repairing injured skin of the present invention is preferably 3.5 to 6 from the standpoint of stability of saccharide andpovidone-iodine. The pH is measured by adding 9 parts by weight of water to 1 part by weight of the composition for repairing injured skin, mixingwell and measuring using a pH meter (e.g. Horiba Ltd: F-24) at 25°C.

Adjustment of pH can be performed by using an acid and an alkali such as hydrochloric acid, citric acid and sodium hydroxide, but the pharmaceutical preparation may be used as a pH buffering system, and lactate buffer, citrate buffer and phosphate buffer may be used.

In the composition for repairing injured skin of the present invention, various other components such as other medicines and pharmaceutically acceptable additives, for example, solubilizing agents, surface active agents and thickener agents may be added in a required amount thereof, so long as an effect of the present invention is not prohibited.

The other medicines include growth factors such as bFGF, EGF, HGF and IGF, and proteins such as silk fibroin, etc.

The solubilizing agents include potassium iodine, sodium iodine, glycerol, polyethyleneglycol(macrogol) 400, polyethyleneglycol(macrogol) 1500, polyethyleneglycol (macrogol) 4000, polyethyleneglycol(macrogol) 6000, polypropyleneglycol, propyleneglycol and dipropyleneglycol, etc.

The surface active agents include poly(oxyethylene)-(105)poly(oxypropylene)(5)glycol, poly(oxyethylene) (120) poly(oxypropylene) (40)glycol, poly(oxyethylene) (160) poly(oxypropylene) (30)glycol, poly(oxyethylene) (196) poly(oxypropylene) (67)glycol, poly(oxyethylene) (20) poly(oxypropylene) (20)glycol, poly(oxyethylene) (200) poly(oxypropylene) (70)glycol, poly(oxyethylene) (3) poly(oxypropylene) (17)glycol, poly(oxyethylene) (42) poly(oxypropylene) (67)glycol, poly(oxyethylene) (54) poly(oxypropylene) (39)glycol, poly(oxyethylene) hydrogenated castor oil, polysorbate, sorbitan monostearate, etc.

The thickener agents include pullulan, sodium carboxymethylcellulose, sodium alginate, povidone, carboxyvinyl polymer, methylcellulose, agar, gelatin, etc.

The composition for repairing injured skin of the present invention can be produced by admixing the components hereinabove and stirring the mixture if necessary under heating to a homogenized state to prepare the ointment state.

The composition for repairing injured skin of the present invention can be preferably used by spreading onto a gauze and attached to lesion. The composition for repairing injured skin of the present invention exhibits superior effects such as absorption of exudation, due to a superior water absorption power thereof.

### Example

The present invention will be explained specifically using examples, but the present invention is not limited by these Examples.

### Example 1

### Production of ointment preparation

### Product of the present invention 1:

A mixture of 9.5172 g of purified water, 0.0828 g of sodium hydroxide, 0.7 g of potassium iodide, 0.1 g of citric acid, 0.2 g of pullulan, 1 g of conc. glycerol, 1 g of 1, 3-butylene glycol, 1g of propylene glycol, 70 g of white soft sugar and 3 g of povidone-iodine was mixed and kneaded well. Then, the mixture was added with 0. 3 gof hydrogenated soybean phospholipid (Lecinol S-10EX, Nikko Chemicals Co.) and kneaded well. Further, the mixture was added with 1 g of macrogol 300, 11 g of macrogol 400, and 1.1 g of poly(oxyethylene) (160) poly (oxypropylene) (30) -glycol dissolved by heating, and kneaded well, and stirred to a homogenized state to produce an ointment preparation (product of the present invention 1).

### Products of the present invention 2 to 5:

Products of the present invention 2 to 4 described in Table 1 were produced by the same procedure as of the product of the present invention 1, and product of the present invention 5 was produced by using soybean lecithin (COATSOMENC-20, NOF) in place of the hydrogenated soybean phospholipid of the product of the present invention 1.

### Comparative product 1:

A mixture of 9.5172 g of purified water, 0. 0828 g of sodium hydroxide, 0.7 g of potassium iodide, 0.1 g of citric acid, 0.2 g of pullulan, 1 g of conc. glycerol, 1 g of 1, 3-butylene glycol, 1g of propylene glycol, 70 g of white soft sugar and 3 g of povidone-iodine was mixed and kneaded well. Further, the mixture was added with 1 g of macrogol 300, 11.3 g of macrogol 400, and 1.1 g of poly(oxyethylene)(160)poly(oxypropylene)(30)glycol dissolved by heating, and kneaded well, and stirred to a homogenized state to produce an ointment preparation (comparative product 1).

About 30 g each of the ointment preparations thus produced were stored in a soft glass bottle (No. 4 standard(JIS) bottle with a plastic inner stopper and a metal cap) in a airtight sealed state at room temperature, then time-dependent changes in consistency, stirring aptitude and extensibility were examined. Results are shown in Table 1.

### (pH)

Each ointment preparation of 1 g collected immediately after production was mixed well with 9 g of water, and pH was measured at 25°C using a pH meter (F-24, Horiba Ltd).

### (Consistency)

Consistency of each ointment preparation was measured immediately after production and after stored for 3 months and 12 months at room temperature.

Using a texture analyzer (TA-XT2i, Stable Micro Systems Inc.), a ball with 1φ cm was penetrated into the ointment preparation by a depth of 2 cm at a rate of 1 mm/s, and a maximum load (g) in the penetration was measured.

### (Stirring aptitude)

Stirring aptitude of each ointment preparation was measured immediately after production and after stored for 3 months and 12 months at room temperature.

Stirring aptitude was evaluated according to the following criteria of four ranks by a sensory examination, after stirring the ointment preparation using a wooden spatula.
ⓞ: very easily stirred, and extremely easy to use;
○: easily stirred, and easy to use;
Δ: difficult to stir, but not necessarily unusable; and
×: very difficult to stir, and unusable.

### (Extensibility)

Extensibility of each ointment preparation immediately after production and after stored for 3 months and 12 months under condition at room temperature were examined.

Extensibility of each ointment preparation was evaluated according to the following criteria of four ranks by sensory examination after stirring the ointment preparation and spreading onto a gauze using a wooden spatula.
ⓞ: very easily extensible, and extremely easy to use;
○: easily extensible, and easy to use;
Δ: difficult to extend, but not necessary unusable; and
×: very difficult to extend, and unusable.

**Table 1**

| Component | | Product of the Present Invention | | | | | Comparative Product |
|---|---|---|---|---|---|---|---|
| | | 1 | 2 | 3 | 4 | 5 | 1 |
| white soft sugar | | 70 | 70 | 70 | 70 | 70 | 70 |
| povidone-iodine | | 3 | 3 | 3 | 3 | 3 | 3 |
| Macrogol 300 | | 1 | 1 | 1 | 1 | 1 | 1 |
| Macrogol 400 | | 11 | 10.8 | 8.3 | 6.3 | 11 | 11.3 |
| conc. Glycerol | | 1 | 1 | 1 | 1 | 1 | 1 |
| 1,3-Butylene glycol | | 1 | 1 | 1 | 1 | 1 | 1 |
| Propylene glycol | | 1 | 1 | 1 | 1 | 1 | 1 |
| Hydrogenated soybean phospholipid | | 0.3 | 0.5 | 3 | 5 | - | - |
| Soybean lecithin | | - | - | - | - | 0.3 | - |
| Poly(oxyethylene)(160) poly(oxypropylene)(30) glycol | | 1.1 | 1.1 | 1.1 | 1.1 | 1.1 | 1.1 |
| Pullulan | | 0.2 | 0.2 | 0.2 | 0.2 | 0.2 | 0.2 |
| Potassium iodide | | 0.7 | 0.7 | 0.7 | 0.7 | 0.7 | 0.7 |
| Citric acid | | 0.1 | 0.1 | 0.1 | 0.1 | 0.1 | 0.1 |
| Sodium hydroxide | | 0.0828 | 0.08 | 0.08 | 0.076 | 0.08 | 0.0828 |
| Purified water | | 9.5172 | 9.52 | 9.52 | 9.524 | 9.52 | 9.5172 |
| pH | (immediately after production) | 4.6 | 4.5 | 4.4 | 4.6 | 4.8 | 4.6 |
| Consistency | immedeately after Production: g | 16.0 | 8.5 | 11.5 | 21.4 | 48.3 | 62.6 |
| | 3 months at room temp.: g | 25.2 | 13.6 | 19.4 | 39.0 | 48.1 | 208.9 |
| | 12 months at room Temp.: g | 30.3 | 14.9 | 21.9 | 32.2 | 45.3 | 254.9 |
| Stirring aptitude | immedeately after Production | ⓞ | ⓞ | ⓞ | ⓞ | ⓞ | ⓞ |
| | 3 months at room temp. | ⓞ | ⓞ | ⓞ | ⓞ | ⓞ | × |
| | 12 months at room Temp. | ⓞ | ⓞ | ⓞ | ⓞ | ⓞ | × |
| Extensibility | immedeately after Production | ⓞ | ⓞ | ⓞ | ⓞ | ⓞ | ⓞ |
| | 3 months at room temp. | ⓞ | ⓞ | ⓞ | ⓞ | ⓞ | ○ |
| | 12 months at room Temp. | ⓞ | ⓞ | ⓞ | ⓞ | ⓞ | Δ |

The ointment preparation containing white soft sugar, povidone-iodine and water, but without containing phospholipid (Comparative product 1) showed significantly increased consistencies and decreased stirring aptitude at the time after stored at room temperature for 3 months. On the other hand, the products of the present invention 1 to 5 showed suppressed increase in consistency after stored at room temperature for 12 months with good stirring aptitudes and extensibilities, and were extremely easy to use.

In addition, white soft sugar (measured by HPLC) and effective amount of iodine (measured by titrimetry) in the products of the present invention were stable after stored at room temperature for 12 months.

## Claims

1. A composition for repairing injured skin comprising 50 to 90% by weight of saccharide, 0.5 to 10% by weight of povidone-iodine, 0.1 to 20% by weight of water and 0.01 to 10% by weight of phospholipid.

2. The composition for repairing injured skin according to claim 1, wherein the saccharide is white soft sugar.

3. The composition for repairing injured skin according to claim 1 or claim 2, wherein the phospholipid is a natural phospholipid or a hydrogenated phospholipid.

4. The composition for repairing injured skin according to any one of claims 1 to 3, wherein pH is 3.5 to 6.
